# EUROPEAN PATENT SPECIFICATION

(11) **EP 3 483 203 B1**
(45) Date of publication and mention of the grant of the patent: **03.09.2025**
(21) Application number: 18184071.1
(22) Date of filing: 17.07.2018
(51) Int. Cl.: C08J 3/22, A61L 27/12, A61L 27/24, D01D 5/098, D01F 4/00, D01F 6/90, D01F 6/92

(54) **COLLAGEN CONTAINING-PLASTIC MASTERBATCH, THE MANUFACTURING METHOD THEREOF, THE PRODUCT THEREFROM, AND APPLICATION THEREOF**
KOLLAGENHALTIGER KUNSTSTOFFMASTERBATCH, HERSTELLUNGSVERFAHREN DAFÜR, DARAUS HERGESTELLTES PRODUKT UND ANWENDUNG DAVON
MÉLANGE MAÎTRE PLASTIQUE CONTENANT DU COLLAGÈNE, SON PROCÉDÉ DE FABRICATION, PRODUIT AINSI FABRIQUÉ ET SON APPLICATION

(30) Priority: 13.11.2017 TW 106139126
(43) Date of publication of application: 15.05.2019
(73) Proprietor: Han Biomedical Inc., New Taipei City 221 (TW)
(72) Inventor: YEN, Hsiao-Cheng, 221 New Taipei City (TW); HUANG, Chun-Hui, 221 New Taipei City (TW)
(74) Representative: Fuchs Patentanwälte Partnerschaft mbB

(56) References cited:
- EP-A1- 3 246 348
- WO-A1-2011/054896
- GB-A- 1 325 271
- JP-A- 2002 030 300
- JP-A- H08 217 933
- JP-A- H1 121 724
- TW-A- 201 809 063
- TETSUYA TAKAHASHI: "Moisture Sorption of Propylene-Ethylene Random Copolymer Fiber Containing Collagen Powder", SEN'I KIKAI GAKKAISHI (JOURNAL OF THE TEXTILE MACHINERY SOCIETY OF JAPAN), vol. 52, no. 10, 25 October 1999 (1999-10-25), pages T195 - T205, XP055506194
- ALBERTO SENSINI ET AL: "Biofabrication of bundles of poly(lactic acid)-collagen blends mimicking the fascicles of the human Achille tendon", BIOFABRICATION, vol. 9, no. 1, 8 March 2017 (2017-03-08), pages 015025, XP055446261, DOI: 10.1088/1758-5090/aa6204
- BURROWS MARIANA C. ET AL: "Hybrid Scaffolds Built From PET and Collagen as a Model For Vascular Graft Architecture", MACROMOLECULAR BIOSCIENCE, vol. 12, no. 12, 15 October 2012 (2012-10-15), DE, pages 1660 - 1670, XP055832573, ISSN: 1616-5187, DOI: 10.1002/mabi.201200154

## Description

### TECHNICAL FIELD

The technical field relates to applications of collagen, and particularly it relates to the application of collagen in artificial fiber and/or plastic-related products.

### BACKGROUND

Collagen mainly exists in the connective tissue of mammals and is the main component of ligaments. It is also the major component of extracellular matrix. Moreover, in addition to being non-toxic to humans, collagen has biodegradable, biocompatible, and low antigenic properties and has excellent effects of stanching bleeding, promoting angiogenesis, promoting wound healing, and preventing wound scar formation. Therefore, collagen has a high value in the fields of medical care and beauty care.

Collagen can be prepared into different types (for example, fibers, plates, sponges, granules, etc.) by cross-linking to making it easy to process with other molecules. However, at present, artificial fibers and/or plastic products containing collagen are mostly obtained by coating or immersing of collagen solutions, and the artificial fibers and/or plastic products containing collagen obtained therefrom have problems with the collagen being easily worn out or lost. Moreover, at present, medical textiles on the market are mainly cotton, polypropylene (PP) and other materials, and there are no biomedical material or medical application textiles developed for bio-functional designs.

Therefore, there is a need for a collagen-containing artificial fiber and/or plastic product in which the collagen will not fall off.

Examples of fibers made from compositions containing a modified polyolefin and collagen are provided in JP H08 217933 A and in the article by Takahashi entitled "Moisture Sorption of Propylene-Ethylene Random Copolymer Fiber Containing Collagen Powder", Journal of the Textile Machinery Society of Japan, vol. 52, no. 10, 25 October 1999 (1999-10-25), pages T195-T205.

The article by Sensini entitled "Biofabrication of bundles of poly(lactic acid)-collagen blends mimicking the fascicles of the human Achille tendon, Biofabrication, vol. 9, no. 1, 8 March 2017, describes polylactic/collagen fibers and non-woven mats, wherein the fibers are obtained by spinning a solution containing collagen type I and polylactic.

WO 2011/054896 A1 discloses a composition, which can be used for making woven and non-woven fabrics or spun bonded fibers, comprising at least one biodegradable aliphatic-aromatic copolyester obtainable starting from mixtures comprising at least one diol, at least one polyfunctional aromatic acid and at least two aliphatic dicarboxylic acids, and at least one polymer of natural origin, which can be collagen.

GB 1 325 271 A discloses fibers obtained by dissolving and decomposing animal fibers, which can be collagen fibers collected from animal leather in an acid solution; adding nylon to the resulting solution with stirring to completely mix and dissolve the nylon therewith; thereafter reducing the solubility of the protein and nylon mixture in the solution thus prepared to precipitate a high molecular bonded product of protein and nylon molecules; and subjecting the high molecular bonded product thus dried to melt spinning.

TW 201 809 063 A discloses a copolyester material with peptide formed by the polymerization of ethylene glycol, collagen, and terephthalic acid, which can be used to spun into fibers for weaving, making clothes, bedding, curtains, sheets and carpets.

JP H11 21724 A discloses a composition to make fibers by melt spinning, and textiles, containing collagen and a modified polyethylene terephthalate, which has a lower melting point to avoid decomposition of the collagen.

The article by Burrows, entitled "Hybrid Scaffolds Built From PET and Collagen as a Model For Vascular Graft Architecture", Macromolecular Bioscience, vol. 12, no. 12, 15 October 2012, pages 1660-1670, describes fibers obtained by electrospinning a solution containing PET and collagen type I.

### SUMMARY

The present invention discloses a collagen containing non-woven fabric, which is made from at least one plastic masterbatch by a meltblowing technique, wherein the at least one plastic masterbatch comprises a collagen containing-plastic masterbatch of which the composition comprises:
a thermoplastic polymer, wherein the thermoplastic polymer is polyethylene terephthalate; and
a collagen, wherein the collagen is uniformly distributed in the thermoplastic polymer,
wherein the collagen containing plastic masterbatch is formed by a melt polymerization method, wherein the melt polymerization method comprises:
   (a) performing a melting procedure on at least one monomer raw material constituting a thermoplastic polymer to obtain a melted monomer raw material; (b) performing a heating and mixing procedure on the melted monomer raw material and a collagen to form a mixture; (c) performing a polymerization reaction on the mixture to form the thermoplastic polymer and make sure that the collagen is uniformly distributed in the thermoplastic polymer to form a collagen-containing polymer; and (d) granulating the collagen-containing polymer to form the collagen containing-plastic masterbatch, wherein in the collagen containing-plastic masterbatch, the content of the collagen is about 1-50 wt%.

In an embodiment of the present invention the non-woven fabric is made from at least one plastic masterbatch, which further comprises a collagen-free plastic masterbatch, wherein composition of the collagen-free plastic masterbatch comprises another thermoplastic polymer. In a particular aspect of this embodiment, the weight ratio of the collagen containing-plastic masterbatch to the collagen-free plastic mastermatch is 1:1-99 and in a further aspect of this embodiment, the thermoplastic polymer and the other thermoplastic polymer are the same.

A further embodiment of the present invention discloses a medical appliance, comprising the collagen containing non-woven fabric. In a particular aspect of this embodiment, the medical appliance comprises a hemostatic gauze or a dressing.

A detailed description is given with reference to the accompanying drawings.

### BRIEF DESCRIPTION OF DRAWINGS

The present invention can be more fully understood by reading the subsequent detailed description and examples with references made to the accompanying drawings, wherein:
FIG. 1 shows a flow process of a melt polymerization (granulation) 100;
FIG. 2 shows a flow process for performing melt spinning with a single screw spinning machine 200;
FIG. 3 shows a standard curve of hydroxyproline concentration versus absorbance value;
FIG. 4 shows the residual collagen contents of the silk stockings containing 1 wt% collagen that have been washed a different number of times;
FIG. 5 shows a photograph of the dyed socks containing 1 wt% of collagen;
FIG. 6 shows a standard curve of hydroxyproline concentration versus absorbance value;
FIG. 7 shows the residual collagen contents of the dyed socks containing 1 wt% collagen that have been washed a different number of times;
FIG. 8 shows a flow process of a melt polymerization (granulation) 100' of;
FIG. 9 shows a flow process of meltblowing for forming non-woven fabric 300;
FIG. 10 shows the photographs of polyethylene terephthalate masterbatches respectively containing 1 wt%, 5 wt%, and 10 wt% collagen obtained in the a process ((A) 1 wt% collagen; (B) 5 wt% collagen; (C) 10 wt% collagen);
FIG. 11 shows the actual collagen contents of the polyethylene terephthalate masterbatches respectively containing 1 wt%, 5 wt%, and 10 wt% collagen obtained in a process; and
FIG. 12 shows bleeding condition of the rat treated by the collagen-polyethylene terephthalate gauze of the present invention and common medicinal gauze at 30th second in a hemostatic efficacy test. (A) Collagen-polyethylene terephthalate gauze: (B) Common medicinal gauze.

### DETAILED DESCRIPTION

In the following detailed description, for purposes of explanation, numerous specific details are set forth in order to provide a thorough understanding of the present invention. It will be apparent, however, that one or more aspects of the present disclosure may be practiced without these specific details. In other instances, well-known structures and devices are schematically shown in order to simplify the drawing.

The present invention provides a collagen containing non-woven fabric, which is made from at least one plastic masterbatch by a meltblowing technique. In the collagen containing-plastic masterbatch mentioned above, the collagen content is about 1-50 wt%, such as 1 wt%, 2 wt%, 5 wt%, 10 wt%, 15 wt%, 20 wt%, 25 wt%, 30 wt%, 35 wt%, 40 wt%, 45 wt%, 50 wt%. A composition of the foregoing collagen containing-plastic masterbatch comprises a thermoplastic polymer and a collagen, wherein the collagen is uniformly distributed in the thermoplastic polymer. By making the collagen in the plastic masterbatch be uniformly distributed in the thermoplastic polymer, the collagen in the plastic product formed by the plastic masterbatch may not be easily damaged due to cleaning or other external forces.

Examples of the thermoplastic polymer can be nylon, polyethylene terephthalate (PET), polypropylene (PP), acrylic acid or spandex. Examples nylon may comprise nylon 6, but they are not limited thereto. Nylon 6 is linear polycaprolactam. Nylon 6 has high mechanical strength, high temperature resistance (HDT=240°C), corrosion resistance (well chemical resistance), high lubricity, low surface friction and high tensile strength and impact strength. According to the present invention, the thermoplastic polymer in the collagen containing-plastic masterbatch mentioned above is polyethylene terephthalate.

Moreover, the type of the collagen in the collagen containing-plastic masterbatch of the present disclosure also has no specific limitation. In one embodiment, the foregoing collagen may have heat resistance to about 260-290°C. Furthermore, in one embodiment, the foregoing collagen may comprise type I collagen.

The foregoing collagen containing-plastic masterbatch of the present invention is formed by a melt polymerization method.

According to the present invention, the foregoing melt polymerization method used for forming the collagen containing-plastic masterbatch comprises the following steps:

First, a melting procedure is performed on at least one monomer raw material constituting the thermoplastic polymer to obtain a melted monomer raw material.

The temperature of the melting procedure may depend on the monomer raw material which is adopted. The temperature of the melting procedure may be about 180-290°C. As an example not according to the present invention, when the foregoing monomer raw material is a monomer raw material of nylon, the temperature of the melting procedure may be about 220-240°C: for example the monomer raw material of nylon can be nylon 6, i.e. caprolactam, and the temperature of the melting procedure may be about 220-240°C, such as about 230°C. According to the present invention, the monomer raw material is a monomer raw material of polyethylene terephthalate, and it may comprise pure terephthalic acid (PTA) and ethylene glycol (EG), and the temperature of the melting procedure may be about 180-290°C, such as about 260°C.

There is no specific limitation for time required for the melting procedure, as long as the monomer raw material can be melted. The time required for the melting procedure may be about 3-10 hours, such as about 3-4 hours, but it is not limited thereto.

Next, a heating and mixing procedure is performed on the foregoing melted monomer raw material and collagen to form a mixture. The weight ratio of the collagen to the at least one monomer raw material is about 1:1-99, such as 1:2, 1:3, 1:4, 1:9, 1:19, 1:99. In one embodiment, the weight ratio of the collagen to the at least one monomer raw material may be about 1:4, 1:9, 1:19 or 1:99.

Furthermore, similarly, the temperature of the foregoing heating and mixing procedure may depend on the monomer raw material which is adopted. The temperature of the foregoing heating and mixing procedure is about 180-290°C. As an example not according to the present invention, when foregoing monomer raw material is a monomer raw material of nylon, the temperature of heating and mixing procedure may be about 180-290°C: for example the monomer raw material of nylon can be nylon 6, i.e. caprolactam, and the temperature of the heating and mixing procedure may be about 220-240°C, such as about 230°C. According to the present invention, the foregoing monomer raw material is a monomer raw material of polyethylene terephthalate, and it may comprise pure terephthalic acid and ethylene glycol, and the temperature of the heating and mixing procedure may be about 180-290°C, such as about 240°C.

In addition, there is not specific limitation for the heating and mixing procedure, as long as the melted monomer raw material and the collagen can be uniformly mixed. The time required for the heating and mixing procedure may be about 3-10 hours, such as about 3-4 hours, but it is not limited thereto.

After that, according to the present invention, a polymerization reaction is performed on a mixture of the melted monomer raw material and the collagen to form the foregoing thermoplastic polymer and to make sure that the collagen is uniformly distributed in the thermoplastic polymer to form a collagen-containing polymer. Similarly, the temperature of the foregoing polymerization reaction may depend on the monomer raw material which is adopted.

In one embodiment, the temperature of the polymerization reaction mentioned above is about 180-290°C. As an example not according to the present invention, when the foregoing monomer raw material is a monomer raw material of nylon, and the temperature of the polymerization reaction may be about 180-290°C: for example the monomer raw material of nylon can be nylon 6, i.e. caprolactam, and the temperature of the polymerization reaction may be about 180-290°C, such as about 260°C. According to the present invention, the foregoing monomer raw material is a monomer raw material of polyethylene terephthalate, and it may comprise pure terephthalic acid and ethylene glycol, and the temperature of the polymerization reaction may be about 180-290°C, such about 260°C.

Similarly, the time required for the polymerization may depend on the monomer raw material which is adopted. The time required for the polymerization may be about 3-20 hours, such as about 10 hours, about 15 hours, but it is not limited thereto.

After that, according to the present invention, a granulation procedure is performed on the obtained collagen-containing polymer to form the collagen containing-plastic masterbatch of the present disclosure. The granulation procedure mentioned above may use any procedure that can divide the obtained polymer into granular finished products and have no specific limitation, for example, a granulation procedure well known in the art can be used. The granulation procedure may be a procedure in which the obtained collagen-containing polymer is cut into granules by a slicer. The granulation procedure may be a procedure in which the obtained collagen-containing polymer is sent to an extruding and mixing granulator and mixed and extruded to form pellets. The particle size of the obtained collagen containing-plastic masterbatch depends on the requirements and has no specific limitation. The particle size of the collagen containing-plastic masterbatch may be about 0.1-3 mm.

In addition, the foregoing melt polymerization method used for forming the collagen containing-plastic masterbatch may further comprise a step of adding water, a polymerization regulator and an external lubricant or adding at least one catalyst to the foregoing melted monomer raw material between performing the foregoing melting procedure and performing the foregoing heating and mixing procedure. The weight ratio of water to the monomer raw material may be about 1:1-99, such as 1:1-20. The weight ratio of water to the monomer raw material may be about 1:4, but it is not limited thereto. The weight ratio of the polymerization regulator to the monomer raw material may be about 1:1-99, such as 1:5-30. The weight ratio of the polymerization regulator to monomer raw material may be about 1:20, but it is not limited thereto. The weight ratio of the external lubricant to the monomer raw material may be about 1:10-200, such as 1:20-200. The weight ratio of the external lubricant to the monomer raw material may be about 1:160, but it is not limited thereto. The weight ratio of the catalyst to the monomer raw material may be about 1:20-150, such as 1:85-110. The weight ratio of the catalyst to the monomer raw material may be about 1:90-100.

The polymerization regulator mentioned above may comprise, but is not limited to, acids, such as acetic acid, adipic acid, or dodecyl mercaptan, etc. Moreover, the external lubricant mentioned above may comprise fatty acid amide, oleamide or stearic acid, etc., but it is not limited thereto. Examples of the at least one catalyst mentioned above may comprise, but are not limited to, antimony trioxide (Sb₂O₃), titanium butoxide (Ti(Obu)₄), antimony acetate (Sb(AC)₃), ethylene glycol antimony (Sb₂(EG)₃) and any combination thereof.

Furthermore, the foregoing melt polymerization method used for forming the collagen containing-plastic masterbatch may further comprise performing an extraction procedure on the obtained collagen containing-plastic masterbatch after the granulation procedure, and performing a drying procedure on the collagen containing-plastic masterbatch after the extraction procedure.

The foregoing extraction procedure is used to remove oligomers, remaining monomers, and/or other impurities in the masterbatch to avoid the oligomers, remaining monomers, etc. in the masterbatch from being oxidized to produce bubbles during the subsequent manufacture of desired plastic products and affecting the quality of the product. The extraction procedure may be performed by adding the obtained collagen containing-plastic masterbatch into hot water, and the temperature of the hot water may be about 80-100°C, but it is not limited thereto. Moreover, since the masterbatch after the extraction procedure will have a higher moisture content, for example, equal to or more than 10%, a drying procedure is performed after the extraction procedure to avoid the masterbatch from subsequent thermal oxidation. The foregoing drying procedure may be performed by drying the collagen containing-plastic masterbatch after the extraction process in a vacuum environment at a temperature of about 80-100°C, such as 100°C.

In addition, the foregoing melt polymerization method used for forming the collagen containing-plastic masterbatch may further comprise the step of adding water, a polymerization regulator and an external lubricant or adding at least one catalyst to the foregoing melted monomer raw material mentioned above between performing the foregoing melting procedure and performing the foregoing heating and mixing procedure, and further comprise the extraction procedure mentioned above and the drying procedure mentioned above after the granulation procedure.

As an example not according to the present invention, in any melt polymerization method used for forming the collagen containing-plastic masterbatch of the present disclosure mentioned above, the at least one monomer raw material constituting the thermoplastic polymer is caprolactam, and the collagen is type I collagen. Moreover, the weight ratio of the type I collagen to the caprolactam may be about 1:1-99, such as about 1:4, 1:5, 1: 19, 1:99, but it is not limited thereto.

Accoding to a specific embodiment of the present invention, in any melt polymerization method used for forming the collagen containing-plastic masterbatch mentioned above, the at least one monomer raw material constituting the thermoplastic polymer comprises pure terephthalic acid and ethylene glycol, and the collagen is type I collagen. Moreover, in this specific embodiment, the weight ratio of the pure terephthalic acid to the ethylene glycol may be about 1:0.1-5, such as about 1:2, but it is not limited thereto. Furthermore, in this specific embodiment, the weight ratio of the type I collagen to the at least one monomer raw material is about 1:1-99, such as about 1:9, 1:19 or 1:99.

Based on the foregoing, the present invention discloses a collagen containing non-woven fabric, which is made from at least one plastic masterbatch by a meltblowing technique, wherein the at least one plastic masterbatch, wherein the least one plastic masterbatch is as disclosed adove.

Furthermore, herein is also disclosed a collagen containing-fiber. The foregoing collagen containing-fiber may be made from at least one plastic masterbatch by a melt spinning technique, and the at least one plastic masterbatch comprises any collagen containing-plastic masterbatch mentioned above, but it is not limited thereto. The collagen content of the collagen containing-fiber may be about 0.04-50 wt%, such as 0.05 wt%, 0.1 wt%, 0.5 wt%, 1 wt%, 2 wt%, 10 wt%, 20 wt%, 50 wt%, but it is not limited thereto.

The "melt spinning technique" described herein may be any melt spinning technique known in the art, for example, after the masterbatch (or polymer) is melted, it is drawn through a spinneret to make it in the form of line, and then is cooled and cured, but the melt spinning technique is not limited thereto. The temperature of the melting mentioned above has no specific limitation, and may depend on the material of the masterbatch, for example, the foregoing temperature of melting may be about 180-290°C. The foregoing temperature of melting may be about 285°C.

The at least one plastic masterbatch mentioned above may be any collagen containing-plastic masterbatch of the present disclosure mentioned above. In an example the thermoplastic polymer of the collagen containing-plastic masterbatch mentioned above is nylon 6. In this specific embodiment, the collagen content of the collagen containing fiber may be about 1-50 wt%, such as 1 wt%, 5 wt%, 10 wt%, 20 wt%, 50 wt%, but it is not limited thereto.

The at least one plastic masterbatch mentioned above may further comprise a collagen-free-plastic masterbatch, and the composition of this collagen-free-plastic masterbatch comprise another thermoplastic polymer. Examples of this another thermoplastic polymer may comprise, but is not limited to nylon, polyethylene terephthalate, polypropylene, acrylic acid and spandex. In the at least one plastic masterbatch mentioned above, the weight ratio of the collagen containing-plastic masterbatch to the collagen-free-plastic masterbatch may be 1:1-24, but it is not limited thereto. In the collagen containing-fiber, the collagen content may be about 0.04-25 wt%, such as 0.05 wt%, 0.1 wt%, 0.5 wt%, 1 wt%, 2 wt%, 10 wt%, 20 wt%, but it is not limited thereto.

The at least one plastic masterbatch mentioned above may further comprise collagen-free-plastic masterbatch, the thermoplastic polymer of the collagen containing-plastic masterbatch and the other thermoplastic polymer of the collagen-free-plastic masterbatch may be the same or different. The thermoplastic polymer of the collagen containing-plastic masterbatch and the other thermoplastic polymer of the collagen-free-plastic masterbatch can be the same. In an example the thermoplastic polymer of the collagen containing-plastic masterbatch and the other thermoplastic polymer of the collagen-free-plastic masterbatch are both nylon, and a monomer raw material constituting the nylon is caprolactam. In the specific example mentioned above, in the collagen containing-fiber, the collagen content may be about 0.04-50 wt%, such as 0.05 wt%, 0.1 wt%, 0.5 wt%, 1 wt%, 2 wt%, 10 wt%, 20 wt%, but it is not limited thereto.

Moreover, herein it is disclosed a textile, which is made from any foregoing collagen containing-fiber of the present disclosure.

The present invention provides a collagen containing non-woven fabric. The foregoing collagen containing non-woven fabric is made from at least one plastic masterbatch by a meltblowing technique, and the at least one plastic masterbatch mentioned above comprises a collagen containing-plastic masterbatch of which the composition comprises:
a thermoplastic polymer, wherein the thermoplastic polymer is polyethylene terephthalate; and
a collagen, wherein the collagen is uniformly distributed in the thermoplastic polymer,
wherein in the collagen containing-plastic masterbatch, a content of the collagen is about 1-50 wt%, and
wherein the collagen containing plastic masterbatch is formed by a melt polymerization method wherein the melt polymerization method comprises:
   (a) performing a melting procedure on at least one monomer raw material constituting the thermoplastic polymer to obtain a melted monomer raw material;
   (b) performing a heating and mixing procedure on the melted monomer raw material and the collagen to form a mixture;
   (c) performing a polymerization reaction on the mixture to form the thermoplastic polymer and make sure that the collagen is uniformly distributed in the thermoplastic polymer to form a collagen-containing polymer; and
   (d) granulating the collagen-containing polymer to form the collagen containing-plastic masterbatch.
The collagen content of the collagen containing non-woven fabric may be about 0.04-50 wt%, such as 0.05 wt%, 0.1 wt%, 0.5 wt%, 1 wt%, 2 wt%, 10 wt%, 20 wt%, 50 wt%, but it is not limited thereto.

The "meltblowing technique" described herein may be any meltblowing technique known in the art, for example, the masterbatch (or polymer) is melted and ejected from a spinneret to make it in the form of net, and then is cooled and cured. The temperature of the melting mentioned above has no specific limitation, and may depend on the material of the masterbatch, for example, the foregoing temperature of melting may be about 180-290°C. The foregoing temperature of melting may be about 260°C.

According to the present invention, the thermoplastic polymer of the collagen containing-plastic masterbatch mentioned above is polyethylene terephthalate and the collagen content of the collagen containing non-woven fabric is about 1-50 wt%, such as 1 wt%, 5 wt%, 10 wt%, 20 wt%, 50 wt%.

In one embodiment of the present invention, the at least one plastic masterbatch mentioned above may further comprise a collagen-free-plastic masterbatch, and the composition of this collagen-free-plastic masterbatch comprise another thermoplastic polymer. Examples of this another thermoplastic polymer may comprise, but is not limited to, nylon, polyethylene terephthalate, polypropylene, acrylic acid and spandex. In the at least one plastic masterbatch mentioned above, the weight ratio of the collagen containing-plastic masterbatch of the present disclosure to the collagen-free-plastic masterbatch may be 1:1-24, but it is not limited thereto. Moreover, in this embodiment, in the collagen containing non-woven fabric, the collagen content may be about 0.04-25 wt%, such as 0.05 wt%, 0.1 wt%, 0.5 wt%, 1 wt%, 2 wt%, 10 wt%, 20 wt%, but it is not limited thereto .

In addition, in the embodiment in which the at least one plastic masterbatch mentioned above may further comprise collagen-free-plastic masterbatch, the thermoplastic polymer of the collagen containing-plastic masterbatch and the other thermoplastic polymer of the collagen-free-plastic masterbatch may be the same or different. In one embodiment, the thermoplastic polymer of the collagen containing-plastic masterbatch and the other thermoplastic polymer of the collagen-free-plastic masterbatch are the same. In one specific embodiment, the thermoplastic polymer of the collagen containing-plastic masterbatch and the other thermoplastic polymer of the collagen-free-plastic masterbatch are both polyethylene terephthalate, and a monomer raw material constituting the polyethylene terephthalate may comprise pure terephthalic acid and ethylene glycol. In the specific embodiment mentioned above, in the collagen containing non-woven fabric, the collagen content may be about 0.04-50 wt%, such as 0.05 wt%, 0.1 wt%, 0.5 wt%, 1 wt%, 2 wt%, 10 wt%, 20 wt%, but it is not limited thereto.

Furthermore, not according to the present invention, it is also disclosed a collagen containing plastic product which is made from at least one plastic masterbatch by a compression molding, calendering, extrusion molding or injection molding technique, and the least one plastic masterbatch described therein is the at least one plastic masterbatch adopted by the collagen containing-fiber or non-woven fabric of the present disclosure mentioned above.

In addition, herein it is also disclosed a medical appliance, commodity or clothing. In a particular embodiment according to the present invention, a medical appliance, comprising the collagen containing non-woven fabric of the present invention is disclosed.

The medical appliance according to the present invention, has the effect of stanching bleeding and/or promoting wound healing because it contains collagen. Examples of the medical appliance mentioned above may comprise, but are not limited to medical gauze (such as hemostatic gauze), various dressings, band-aid (such as the gauze part of the band-aid), surgical suture. In a particular aspect of the present invention the medical appliance comprises a hemostatic gauze or a dressing. Moreover, examples of the commodity may comprise a non-woven facial mask, a sanitary pad, a diaper, but they are not limited thereto. Examples of clothing may comprise underwear, corset, girdle, silk stockings, but they are not limited thereto. The haemostatic gauze formed with the collagen fiber or non-woven fabric has excellent hemostatic effect.

Although collagen has biodegradable, biocompatible, and low antigenic properties and has excellent effects of stanching bleeding, promoting angiogenesis, promoting wound healing, and preventing wound scar formation, if only collagen dressings are used as general wounds dressings, there will be a problem of excessive cost. In the present invention, collagen and polyethylene terephthalate, are polymerized to form, for example, collagen-polyethylene terephthalate gauze which can adsorb blood and tissue fluid. Moreover, because polyethylene terephthalate, have properties of smooth surfaces and small pores, they are not susceptible to bacterial growth. Therefore, gauze of the present invention can achieve hemostatic effect, and will not cause sticking to the wound. At the same time, it can reduce production costs, and also increase the value of collagen and plastics in medical appliance field and clinical application.

### EXAMPLES

### Reference Example 1: Collagen-containing nylon masterbatch and products thereof

### Reference Example 1-1: Preparation of collagen-containing nylon 6 masterbatch

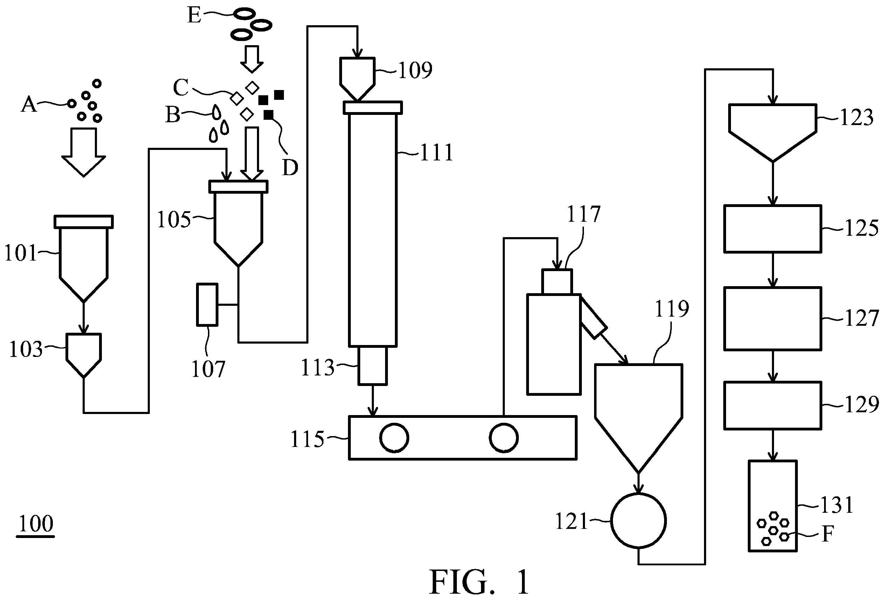

FIG. 1 shows a flow process of a melt polymerization (granulation) 100. Preparation of collagen-containing nylon 6 masterbatch of this example can be referred to FIG. 1 and interpreted as follows:
1. 20 kg of caprolactam crystallized raw material A was added into a melting tank 101, and a melting procedure was performed on the caprolactam to make sure that the caprolactam is in a molten state, in which the melting temperature was set to 230° C, and the melting time was 3-4 hours.
2. After the caprolactam in a molten state was filtered by a filter 103, 5 kg of water B, 1 kg of acetic acid (polymerization regulator) C, and 0.125 kg of oleamide (external lubricant) D were added to the caprolactam in a molten state in a monomer storage tank 105 and uniformly mixed with the caprolactam in a molten state to form a first mixture.
3. Next, 5 kg of collagen raw material E was added to the monomer storage tank 105 and uniformly mixed with the foregoing first mixture and heated to 230°C (about 3-4 hours; the pressure was 1800 kg/cm² ) to form a second mixture.
4. After that, the second mixture is fed to the polymerization tank 111 through the pre-polymerizer 109 by a pump 107 to perform solid-phase polymerization, wherein the polymerization temperature is 260°C and the polymerization pressure is 2000 kg/cm².
5. During the polymerization, the terminal amino group of the caprolactam molecule undergoes a condensation reaction with the carboxyl group through high-temperature drying, thereby generating a polymer with a high degree of polymerization containing uniformly dispersed collagen. After 15 hours of retention, polymerization equilibrium was reached.
6. The obtained polymer containing uniformly dispersed collagen is transported to a slicer 117 through a front end of injection nozzle 113 and an belt conveyer 115 to be cut into granular form, and total 20 kg of collagen containing nylon 6 masterbatches (the collagen content was 20%) were obtained.
7. The obtained collagen containing nylon 6 masterbatches were transported to an extraction equipment 125 through a slice storage barrel 119, a blower 121, and a separator 123 to perform an extraction procedure. In the extraction procedure, the collagen containing nylon 6 masterbatches were added into hot water at 100°C for extraction to remove impurities (10% caprolactam and 2-3% cyclic oligomers).
8. After the extraction procedure, since the collagen containing nylon 6 masterbatches contained equal to or more than 10% moisture, the collagen containing nylon 6 masterbatches were transported to a vacuum dryer 129 through a wet slice storage barrel 127 to perform a drying procedure to remove moisture. In the drying procedure, the collagen containing nylon 6 masterbatches were vacuum-dried at a temperature of 100°C.
9. After the drying procedure, the dried the collagen containing nylon 6 masterbatches F were transported to a dry slice storage barrel 131 for storage to complete the preparation of the collagen containing nylon 6 masterbatches.

### Reference Example 1-2: Preparation of yarn of collagen-containing nylon 6:

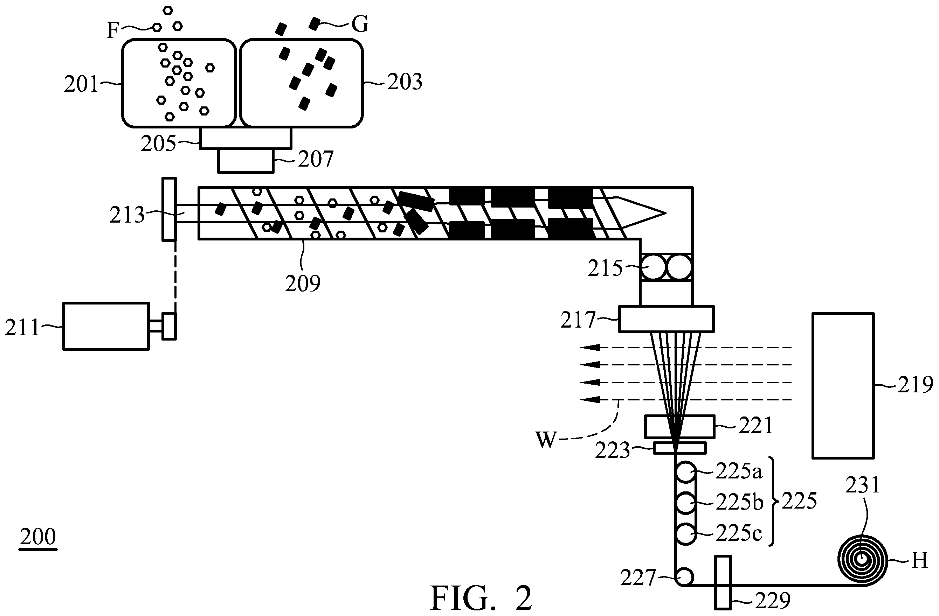

FIG. 2 shows a flow process for performing melt spinning with a single screw spinning machine 200. Preparation of yarn of collagen containing nylon 6 of this example can be referred to FIG. 2 and interpreted as follows:
1. 2 kg of the above-obtained collagen containing nylon 6 masterbatches F and 38 kg of the normal nylon 6 masterbatches G were respectively added into a first feeding tank 201 and a second feeding tank 203 to make sure that the resulting yarn has 1 wt % of collagen.
2. The two kinds of masterbatches were transported to a spinning machine body 209 through a metering tank 205 and a mixing tank 207 and heated to 285°C to make sure the two kinds of masterbatches are in a molten state and form a melt. The parameters for spinning were set as follows:
   Denier: 70;
   Spun: 72;
   Spinning rate: 4000 m/minute.
3. The screw 213 configured in the spinning machine body 209 was pushed by the motor 211 to further evenly mix the melt and the melt was quantified by a quantitative pump 215 and ejected through a spinneret at the front end of a spinneret set 217. The melt ejected from the spinneret passed through the cooling wind W which was released from a cooling cabinet 219 and a cooling and curing procedure was started to form a fibrous plastic.
4. During the curing procedure, the fibrous plastic was guided to a roller group 225 through a nozzle 221 and a wire guide 223 and stretched by three rollers 225a, 225b and 225c in the roller group 225 to make sure that the molecular chain produces a forward arrangement and crystallization and give the fiber strength. The parameters of the three rollers 225a, 225b, and 225c are as follows:
   Roller 225a: Surface temperature was 28°C; rotational speed (rpm) was 425;
   Roller 225b: Surface temperature was 50°C; rotational speed (rpm) was1600;
   Roller 225c: Surface temperature was 80°C; rotational speed (rpm) was1600.
5. In a condition of that the fiber was not curled, the fiber was collected by a winder 231 through an upper reel 227 and a separator plate 229 as a single coil yarn H (24.4 kg of yarn).

### Reference Example 1-3: Preparation of textile

### 1. Silk stockings

The 1 wt% collagen-containing yarn obtained above was woven into silk stockings to obtain silk stockings containing 1 wt% collagen (Denier: 70; Spun of yarn: 72).

### 2. Dyed socks

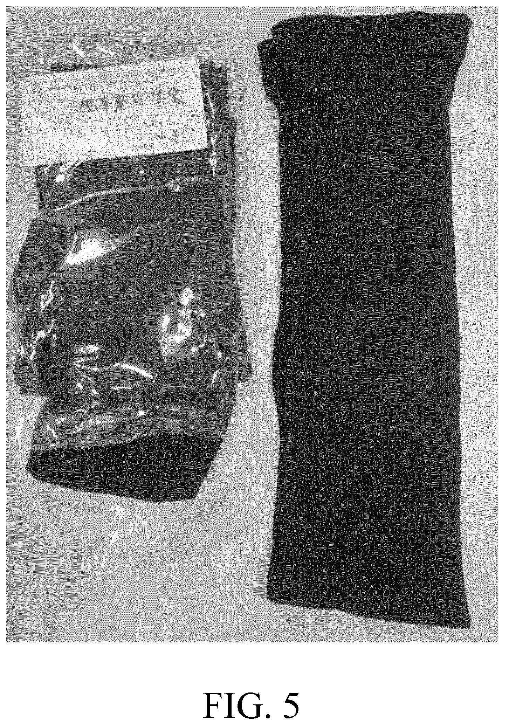

The 1 wt% collagen-containing yarn obtained above was dyed and then woven into socks to obtain dyed socks containing 1 wt% of collagen (Denier: 70; Spun of yarn: 72) (referring to FIG. 5).

### Reference Example 1-4: Test of water washing resistance of collagen-containing textile of the present disclosure

### Reference Example 1-4-1: Test of water washing resistance of silk stockings

### A. Method

### 1. Water washing for silk stocking sample

The silk stockings were water-washed with an operator wearing a plastic glove to perform the usual hand wash. The silk stockings were immersed in 50 ml of pure water (ddH₂O) to wash by hand, and then the silk stocking samples were squeezed to dry. For different silk stocking samples, the above steps were repeated for different times to obtain silk stocking samples with different number of times for washing (0 time, 1 time, 5 times, 10 tomes, 20 times, and 50 times). After that, the silk stocking samples were vacuum dried at 55°C.

### 2. Acid hydrolysis treatment for silk stocking sample

0.25 g of the dried silk stocking sample mentioned above was placed in a test tube and 0.5 ml of sulfuric acid (H₂SO₄; 18 M) was added therein. After that, the opening of the tube was sealed and the tube was heated at 110°C for 24 hours. After heating, the test tube was placed at room temperature for cooling. Then, 1.5 ml of sodium hydroxide (NaOH; 12 M) was added to the tube and mixed evenly with the contents in the tube. Next, 400 µl of liquid was taken from the tube to a new tube. The new tube was vacuum dried at 55°C for 24 hours. Then, 3 ml of 99% alcohol was added to this test tube and mixed evenly with the contents in this tube. 600 µl of liquid was taken from this tube to another new tube and vacuum dried for 24 hours. After that, the dry distillated sample was added to 3 ml of citric acid phosphate buffer (pH=5.5) (720 times dilution) to obtain a treated sample to be tested.

### 3. Quantification of collagen contained in the yarn of silk stocking

Since there is currently no standard method for quantifying collagen contained in yarn, in the present disclosure, a method for determining the content of the collagen in the yarn by determining the hydroxyproline (Hyp) content is developed.

Hydroxyproline is the major component of collagen, and thus the collagen content in the sample can be represented by the content of hydroxyproline.

Hydroxyproline standard sample solutions at concentrations of 0, 0.0625, 0.125, 0.25, 0.5, 1, 2, 4, 8, and 16 µg/ml were prepared. Different concentrations of hydroxyproline standard sample solution and test sample were respectively taken 200 µl and added into different wells of a 96-well plate. After that, 750 µl/well of chloramine T was added to the 96-well plate and reacted at room temperature for 15 minutes. Then, 750 µl/well of Ehrlich's reagent (dimethylaminobenzaldhyde, DAMB) was further added to the 96-well plate and reacted at 75°C for 16 minutes. Finally, the absorbance at 540 nm (OD₅₄₀) of different concentrations of hydroxyproline standard sample solutions and the sample to be tested was determined by an ELISA reader.

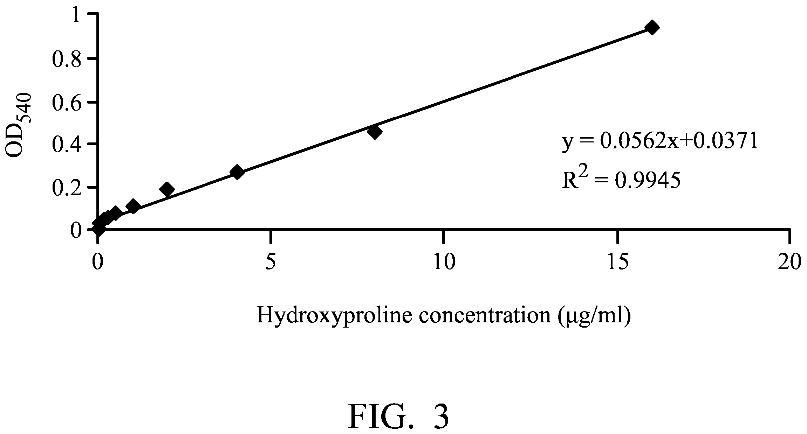

According to the absorbance values at 540 nm (OD₅₄₀) corresponding to different concentrations of hydroxyproline, a standard curve of hydroxyproline concentration versus absorbance value of was plotted (referring to FIG. 3). Moreover, based on the standard curve, the determined absorbance at 540 nm (OD₅₄₀) of the sample to be tested was converted into the hydroxyproline content of the sample to be tested to further determine the collagen content of the sample to be tested.

### B. Result

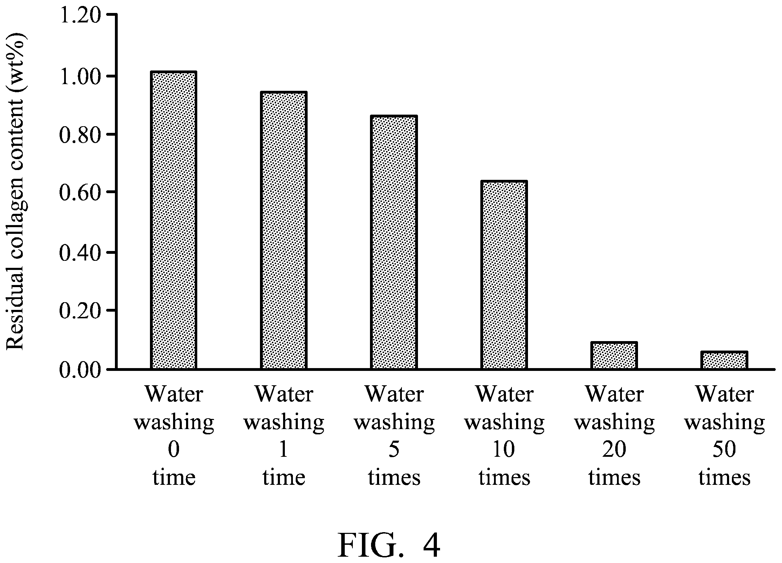

The residual collagen contents of the silk stocking samples that have been washed a different number of times (0, 1, 5, 10, 20, and 50 times) were measured by the above method to evaluate the water washing resistance of the silk stocking containing collagen. The results are shown in Table 1 and FIG. 4.

**Table 1: The residual collagen contents of the silk stocking containing 1 wt% collagen that have been washed a different number of times**

| Number of times for water washing | OD₅₄₀ | Collagen content (wt%) |
|---|---|---|
| 0 | 0.121 | 1.01 |
| 1 | 0.101 | 0.94 |
| 5 | 0.095 | 0.86 |
| 10 | 0.076 | 0.64 |
| 20 | 0.056 | 0.10 |
| 50 | 0.049 | 0.06 |

Tables 1 and FIG. 4 clearly show that after being water-washed 10 times, the silk stocking woven with the 1 wt% collagen-containing yarn of the present disclosure could still retain up to 0.60 wt% or more of collagen. Moreover, even after 50 washes with water, silk stocking woven with the 1 wt% collagen-containing yarn of the present disclosure could still maintain 0.06 wt% of the collagen.

Accordingly, it is known that a product woven with the 1 wt% collagen-containing yarn of the present disclosure has extremely excellent water washing resistance.

### Reference Example 1-4-2: Test of water washing resistance of dyed socks

### A. Method

### 1. Water washing for dyed sock sample

The water washing method for the dyed sock samples was the same as the washing method described in "1. Water washing for silk stocking sample" of "A. Method" of "Reference Example 1-4-1: Test of water washing resistance of silk stockings".

### 2. Acid hydrolysis treatment for dyed sock sample

The method for acid hydrolysis treatment for dyed sock sample samples was the same as the treat method described in "2. Acid hydrolysis treatment for silk stocking sample" of "A. Method" of "Reference Example 1-4-1: Test of water washing resistance of silk stockings".

### 3. Quantification of collagen contained in the yarn of dyed sock sample

The quantifying method for collagen contained in the yarn of dyed sock sample can be referred to the quantifying method recited in "3. Quantification of collagen contained in the yarn of silk stocking sample" of "A. Method" of "Reference Example 1-4-1: Test of water washing resistance of silk stockings".

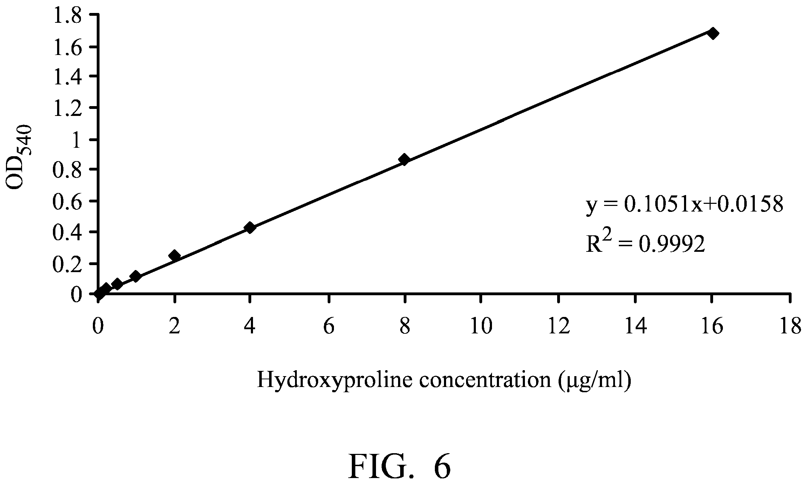

According to the absorbance values at 540 nm (OD₅₄₀) corresponding to different concentrations of hydroxyproline, a standard curve of hydroxyproline concentration versus absorbance value of was plotted (referring to FIG. 6). Moreover, based on the standard curve, the determined absorbance at 540 nm (OD₅₄₀) of the sample to be tested was converted into the hydroxyproline content of the sample to be tested to further determine the collagen content of the sample to be tested.

### B. Result

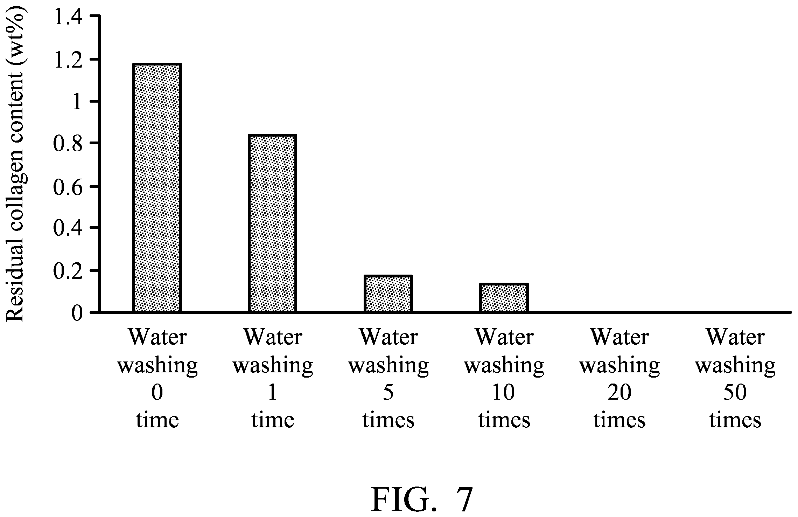

The residual collagen contents of the dyed sock samples that have been washed a different number of times (0, 1, 5, 10, 20, and 50 times) were measured by the above method to evaluate the water washing resistance of the dyed sock containing collagen. The results are shown in Table 2 and FIG. 7.

**Table 2: The residual collagen contents of the dyed sock containing 1 wt% collagen that have been washed a different number of times**

| Number of times for water washing | OD₅₄₀ | Collagen content (wt%) |
|---|---|---|
| 0 | 0.065 | 1.179 |
| 1 | 0.048 | 0.844 |
| 5 | 0.023 | 0.181 |
| 10 | 0.022 | 0.143 |
| 20 | 0.011 | <0.01 |
| 50 | 0.001 | <0.01 |

Tables 2 and FIG. 7 clearly show that after being water-washed 10 times, the dyed sock woven with the 1 wt% collagen-containing yarn of the present disclosure could still retain up to 0.14 wt% or more of collagen.

Accordingly, it is known that a product woven with the 1 wt% collagen-containing yarn of the present disclosure has excellent water washing resistance.

### Example 2: Collagen-containing polyethylene terephthalate masterbatch and products thereof

### Reference Example 2-1: Preparation of collagen-containing polyethylene terephthalate masterbatch

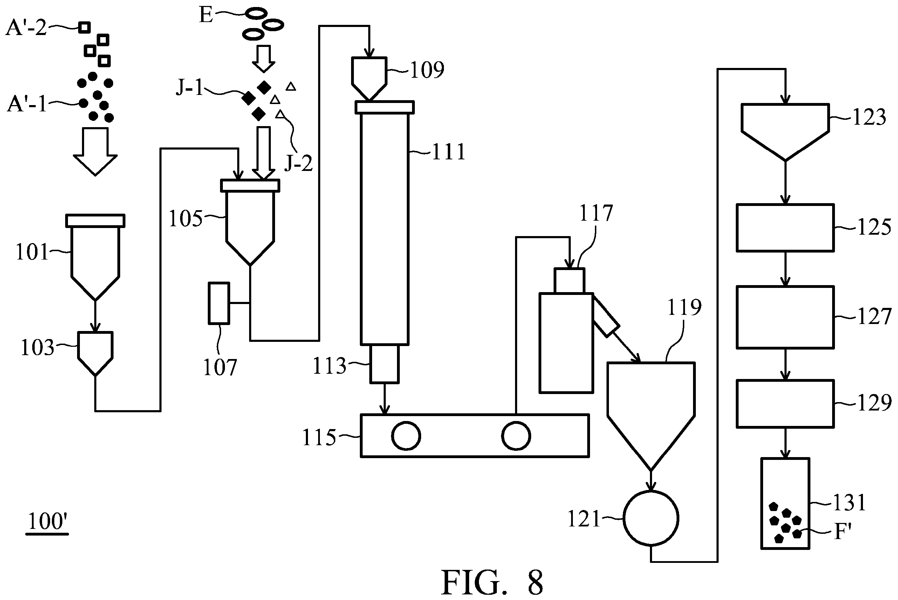

FIG. 8 shows a flow process of a melt polymerization (granulation) 100'. Preparation of collagen-containing polyethylene terephthalate masterbatch of this example can be referred to FIG. 8 and interpreted as follows:
1. Pure terephthalic acid (PTA) raw material A'-1 and ethylene glycol (EG) raw material A'-2 (13.2 kg of pure terephthalic acid and 6.6 kg of ethylene glycol, 12.6 kg of pure terephthalic acid and 6.33 kg of ethylene glycol, or 12 kg of pure terephthalic acid and 6 kg of ethylene glycol) were added into a melting tank 101, and a melting procedure was performed on the pure terephthalic acid and ethylene glycol to make sure the pure terephthalic acid and ethylene glycol are in a molten state, in which the melting temperature was set to 260° C, and the melting time was 4 hours.
2. After the pure terephthalic acid and ethylene glycol in a molten state was filtered by a filter 103, 0.1 kg antimony trioxide (Sb₂O₃) (catalyst) J-1 and 0.1 kg titanium butoxide (Ti(Obu)₄)(catalyst) J-2 were added to the pure terephthalic acid and ethylene glycol in a molten state in a monomer storage tank 105 and uniformly mixed with the pure terephthalic acid and ethylene glycol in a molten state to form a first mixture.
3. Next, collagen raw material E (0.2 kg, 1 kg or 2 kg) was added to the monomer storage tank 105 (the total weight of collagen, pure terephthalic acid raw material and ethylene glycol raw material was about 20 kg) and uniformly mixed with the foregoing first mixture and heated to 240°C (about 4 hours; the pressure was 1500-2000 kg/cm²) to form a second mixture.
4. After that, the second mixture is fed to the polymerization tank 111 through the pre-polymerizer 109 by a pump 107 to perform solid-phase polymerization, wherein the polymerization temperature is 260°C and the polymerization pressure is 2000 kg/cm².
5. During the polymerization, the terminal amino group of the pure terephthalic acid and ethylene glycol molecule undergoes a condensation reaction with the carboxyl group through high-temperature drying, thereby generating a polymer with a high degree of polymerization containing uniformly dispersed collagen. After 10 hours of retention, polymerization equilibrium was reached.
6. The obtained polymer containing uniformly dispersed collagen is transported to a slicer 117 through a front end of injection nozzle 113 and a belt conveyer 115 to be cut into granular form, and collagen-containing masterbatches (the collagen content was 1 wt%, 5 wt% or 10 wt%) were obtained.
7. The obtained collagen containing polyethylene terephthalate masterbatches were transported to an extraction equipment 125 through a slice storage barrel 119, a blower 121, and a separator 123 to perform an extraction procedure. In the extraction procedure, the collagen containing polyethylene terephthalate masterbatches were added into hot water at 100°C for extraction to remove impurities.
8. After the extraction procedure, since the collagen containing polyethylene terephthalate masterbatches contained equal to or more than 10% moisture, the collagen containing polyethylene terephthalate masterbatches were transported to a vacuum dryer 129 through the wet slice storage barrel 127 to perform a drying procedure to remove moisture. In the drying procedure, the collagen containing polyethylene terephthalate masterbatches were vacuum-dried at a temperature of 100°C.
9. After the drying procedure, the dried the collagen containing polyethylene terephthalate masterbatches F were transported to a dry slice storage barrel 131 for storage to complete the preparation of the collagen containing polyethylene terephthalate masterbatches.

### Example 2-2 (according to the present invention): Preparation of non-woven fabric of collagen-containing polyethylene terephthalate

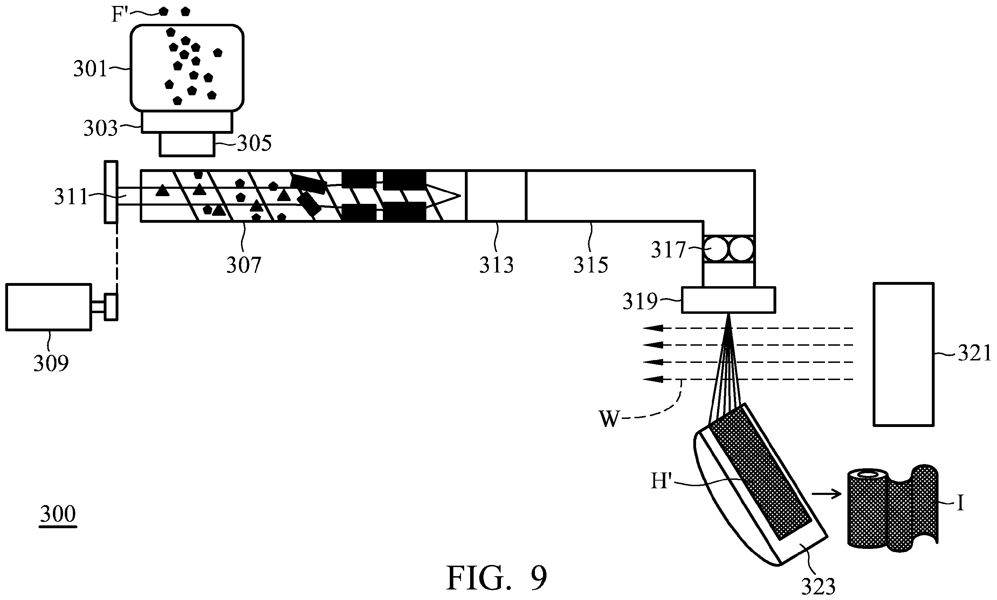

FIG. 9 shows a flow process of meltblowing for forming non-woven fabric 300. Preparation of polyethylene terephthalate non-woven fabric containing collagen of this example can be referred to FIG. 9 and interpreted as follows:
1. The above-obtained collagen containing polyethylene terephthalate masterbatches F' were added into a feeding tank 301 to make sure the resulting non-woven fabric has 1 wt % of collagen.
2. The masterbatches were transported to an extruder 307 through a metering tank 303 and the mixing tank 305 and the heating was started (initial temperature was 120°C) to put the masterbatches into a molten state and form a melt. The screw 311 configured in the extruder 307 was pushed by the motor 309 to further evenly mix the melt, and the melt flow index was set to 50. After that, the temperature was continuously raised and the melt entered into an extruder 315 through a filter device 313, and quantified by the quantitative pump 317. When the temperature was raised to 210°C, the melt was ejected through a spinneret at the front end of a spinneret set 319. The parameters for meltblowing were set as follows:
   Initial temperature: 120°C;
   Ejecting initial temperature: 210°C;
   Spinneret diameter: 0.05 cm;
   Melt flow: 0.5 g;
   Flow rate control: 2 m/s;
   Non-woven thickness: 1 mm.
4. The melt ejected from the spinneret passed through the cooling wind W which was released from a cooling cabinet 321 to make the melt on a conveyor belt 323 be cooled and cured to be in the form of net to form a semi-finished product of non-woven fabric.
5. The non-woven semi-finished product on conveyor belt 323 was thermal-cured to form nonwoven fabric H'.
6. Finally, the nonwoven fabric H' was separated from the conveyor belt 323 and wound up to form a single roll nonwoven fabric I.

### Reference Example 2-3 : Determination of actual collagen content of collagen-containing polyethylene terephthalate masterbatch

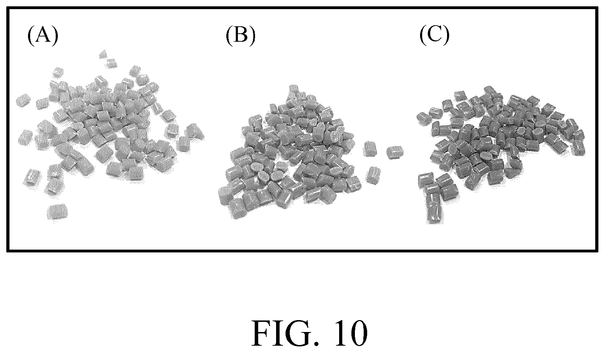

FIG. 10 shows the photographs of polyethylene terephthalate masterbatches obtained in the above process respectively containing 1 wt%, 5 wt%, and 10 wt% collagen ((A) 1 wt% collagen; (B) 5 wt% collagen; (C) 10 wt% collagen).

### A. Method

### 1. Acid hydrolysis treatment for sample of collagen-containing polyethylene terephthalate masterbatch

0.25 g of polyethylene terephthalate masterbatches containing 1 wt%, 5 wt% or 10 wt% collagen were placed in a test tube and 0.5 ml of sulfuric acid (H₂SO₄; 18 M) was added therein. After that, the opening of the tube was sealed and the tube was heated at 110°C for 24 hours. After heating, the test tube was placed at room temperature for cooling. Then, 1.5 ml of sodium hydroxide (NaOH; 12 M) was added to the tube and mixed evenly with the contents in the tube. Next, 400 µl of liquid was taken from the tube to a new tube. The new tube was vacuum dried at 55°C for 24 hours. Then, 3 ml of 99% alcohol was added to this test tube and mixed evenly with the contents in this tube. 600 µl of liquid was taken from this tube to another new tube and vacuum dried for 24 hours. After that, the dry distillated sample was added to 3 ml of citric acid phosphate buffer (pH=5.5) (720 times dilution) to obtain a treated sample to be tested.

### 2. Quantification of collagen contained in the collagen-containing polyethylene terephthalate masterbatch

Samples to be tested were respectively taken 200 µl and added into different wells of a 96-well plate. After that, 750 µl/well of chloramine T was added to the 96-well plate and reacted at room temperature for 15 minutes. Then, 750 µl/well of Ehrlich's reagent (dimethylaminobenzaldhyde, DAMB) was further added to the 96-well plate and reacted at 75°C for 16 minutes. Finally, the absorbance at 540 nm (OD₅₄₀) of different concentrations of hydroxyproline standard sample solutions and the sample to be tested was determined by an ELISA reader.

Based on the standard curve shown in FIG. 6, the determined absorbance at 540 nm (OD₅₄₀) of the sample to be tested was converted into the hydroxyproline content of the sample to be tested to further determine the collagen content of the sample to be tested.

### B. Result

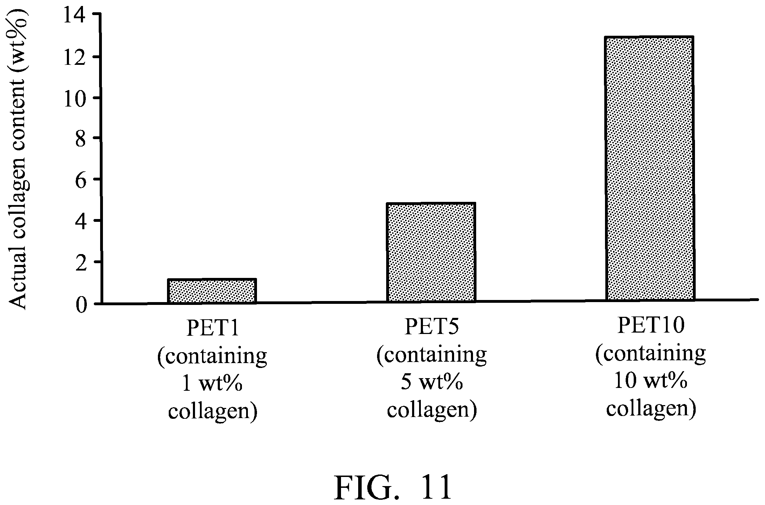

The actual collagen contents of the above-obtained polyethylene terephthalate masterbatches containing 1 wt%, 5 wt%, and 10 wt% collagen were determined by the foregoing method. The results are shown in FIG. 11 and Table 3.

**Table 3: Actual collagen contents of the prepared polyethylene terephthalate masterbatches containing 1 wt%, 5 wt%, and 10 wt% collagen**

| Sample | OD₅₄₀ | Actual collagen content (wt%) |
|---|---|---|
| Polyethylene terephthalate masterbatches containing 1 wt% | 0.042 | 1.16 |
| Polyethylene terephthalate masterbatches containing 5 wt% | 0.134 | 4.75 |
| Polyethylene terephthalate masterbatches containing 10 wt% | 0.296 | 12.77 |

Table 3 and FIG. 11 clearly show that masterbatches containing the three collagen contents produced by the process of the present disclosure have actual collagen contents similar to the theoretical collagen contents thereof. In the polyethylene terephthalate masterbatch containing 1 wt% and 10 wt% collagen, the actual collagen contents were even higher than the theoretical collagen contents. Therefore, it is known that the process of the present disclosure is a stable process that is able to effectively produce plastic masterbatches with a predetermined collagen content.

### Example 2-4 (according to the present invention): Hemostatic efficacy test for collagen-polyethylene terephthalate gauze (collagen-containing polyethylene terephthalate non-woven fabric)

### A. Method

Six Sprague-Dawley female rats used in the experiment were purchased from BioLASCO Taiwan Co., Ltd., and body weights of the rats were between 200 g and 250 g. The rats were divided into two groups which were one control group and one experimental group while there were 3 rats in each group. The collagen-polyethylene terephthalate gauze obtained by the above process was used as the testing gauze in the experimental group while commercial collagen-free polyethylene terephthalate nonwoven fabric (China Surgical Dressings Center Co., Ltd, medicinal gauze-sterilized non-woven mat (material: polyethylene terephthalate, Rayon)) was used as the testing gauze in the control group. All experiments were performed by the same person.
1. The rats were anesthetized by injection of Zoletil (0.1 ml/100 g body weight) and Xylazine (Rompun) (0.05 ml/100 g body weight).
2. After anesthesia, the right femoral artery of the rat was perforated with a 24 gauge needle. Next, the testing gauze (4x4 cm²) was placed at the bleeding site of the blood vessel and pressurized. 30 seconds after arterial bleeding, the timing began. Every 30 seconds, the hemostatic efficacy was record and if bleeding was stanched, the test was ended. If bleeding continued, the bleeding site was pressurized for further 30 seconds and hemostatic efficacy was re-evaluated. If the bleeding still occurred after 90 seconds of pressurization, the test was determined to be invalid. Heat lamp was used throughout the experiment to ensure the rats maintain normal body temperature at 37±0.5°C and the rat body temperature was recorded.
3. At the end of the experiment, all animals were sacrificed with carbon dioxide inhalation.

### B. Result

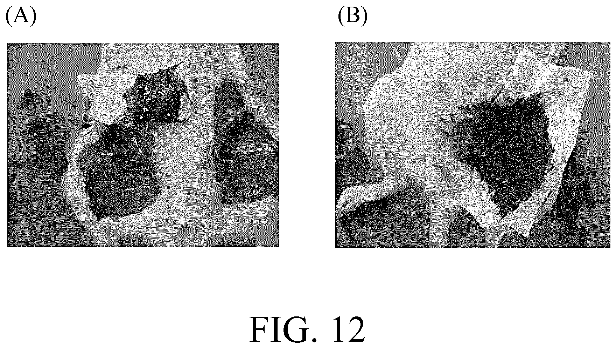

Hemostatic efficacy evaluation for the collagen-polyethylene terephthalate gauze of the present disclosure and common PET gauze was performed by the foregoing method. The results are shown in Table 4 and FIG. 12 ((A) Experimental group: collagen-polyethylene terephthalate gauze; (B) Control group: medicinal gauze).

**Table 4**

| Experimental group | Bleeding stanching time (second) | | | |
|---|---|---|---|---|
| Collagen-polyethylene terephthalate gauze | Rat 1 | Rat 2 | Rat 3 | Mean ± standard deviation |
| | 46 | 50 | 63 | 53.00±8.88 |

| Control group | Bleeding stanching time (second) | | | |
|---|---|---|---|---|
| Common medicinal gauze | Rat 1 | Rat 2 | Rat 3 | Mean ± standard deviation |
| | 125 | 145 | 143 | 137.66±11.01 |

FIG. 12 shows bleeding condition of the rat at 30th second in each group. According to FIG. 12, it is known that in the experimental group, at the 30th second, a blood clot was produced at the site of arterial trauma in the rat, and coagulation occurred. In contrast, in the control group, at the 30th second, no blood clot was produced at the site of arterial trauma in the rat.

The bleeding stanching time of rats in the experimental group and the control group shown in Table 4 were statistically analyzed with student's t-test, and the p value was 0.0037, and thus it was confirmed that statistically significant differences between the experimental group and the control group. Specifically, compared to the commercially medicinal gauze, the collagen-polyethylene terephthalate gauze of the present disclosure can stanch bleeding in a shorter time.

In addition, in the literature, Koksal, O., Ozdemir, F., Cam Etoz, B., & Isbil, N. (2011). Ulus Travma Acil Cerrahi Derg, 17(3), 199-204., an object with a bleeding stanching time of less than 90 seconds is defined as that it has a hemostatic effect. Since a bleeding stanching time for the collagen-polyethylene terephthalate gauze of the present disclosure is 53.00±8.88 seconds, it is determined that the collagen-polyethylene terephthalate gauze of the present disclosure has a hemostatic effect.

Accordingly, the above mentioned tests have confirmed that the collagen-polyethylene terephthalate gauze of the present disclosure has an excellent hemostatic effect and can be used for medical hemostasis.

It will be apparent to those skilled in the art that various modifications and variations can be made to the disclosed embodiments. It is intended that the specification and examples be considered as exemplary only, with the true scope of the present invention being indicated by the following claims.

## Claims

1. A collagen containing non-woven fabric, which is made from at least one plastic masterbatch by a meltblowing technique, wherein the at least one plastic masterbatch comprises a collagen containing-plastic masterbatch of which the composition comprises:
a thermoplastic polymer, wherein the thermoplastic polymer is polyethylene terephthalate; and
a collagen, wherein the collagen is uniformly distributed in the thermoplastic polymer,
wherein in the collagen containing-plastic masterbatch, a content of the collagen is about 1-50 wt%, and
wherein the collagen containing-plastic masterbatch is formed by a melt polymerization method, wherein the melt polymerization method comprises:
(a) performing a melting procedure on at least one monomer raw material constituting the thermoplastic polymer to obtain a melted monomer raw material;
(b) performing a heating and mixing procedure on the melted monomer raw material and the collagen to form a mixture;
(c) performing a polymerization reaction on the mixture to form the thermoplastic polymer and make sure that the collagen is uniformly distributed in the thermoplastic polymer to form a collagen-containing polymer; and
(d) granulating the collagen-containing polymer to form the collagen containing-plastic masterbatch.

2. The collagen containing non-woven fabric as claimed in claim 1, wherein the at least one plastic masterbatch further comprises a collagen-free plastic masterbatch, wherein composition of the collagen-free plastic masterbatch comprises another thermoplastic polymer.

3. The collagen containing non-woven fabric as claimed in claim 2, wherein a weight ratio of the collagen containing-plastic masterbatch as claimed in claim 1 to the collagen-free plastic masterbatch is 1:1-99.

4. The collagen containing non-woven fabric as claimed in claim 2, wherein the thermoplastic polymer and the other thermoplastic polymer are the same.

5. A medical appliance, comprising the collagen containing non-woven fabric as claimed in claim 1 or 2.

6. The medical appliance as claimed in claim 5, wherein the medical appliance comprises a hemostatic gauze or a dressing.

## Patentansprüche

1. Kollagenhaltiger Vliesstoff, der aus zumindest einem Kunststoffmasterbatch durch eine Meltblown-Technik hergestellt ist, wobei das zumindest eine Kunststoffmasterbatch das kollagenhaltige Kunststoffmasterbatch umfasst, wobei die Zusammensetzung umfasst:
ein thermoplastisches Polymer, wobei es sich bei dem thermoplastischen Polymer um Polyethylenterephthalat handelt; und
ein Kollagen, wobei das Kollagen im thermoplastischen Polymer gleichmäßig verteilt ist, wobei in dem kollagenhaltigen Kunststoffmasterbatch ein Gehalt des Kollagens etwa 1-50 Gew.-% beträgt, und
wobei das kollagenhaltige Kunststoffmasterbatch durch ein Schmelzpolymerisationsverfahren gebildet wird, wobei das Schmelzpolymerisationsverfahren umfasst:
(a) Durchführen eines Schmelzverfahrens an zumindest einem Monomer-Rohmaterial, aus dem das thermoplastische Polymer besteht, um ein geschmolzenes Monomer-Rohmaterial zu erhalten;
(b) Durchführen eines Erwärmungs- und Mischverfahrens an dem geschmolzenen Monomer-Rohmaterial und dem Kollagen, um ein Gemisch zu bilden;
(c) Durchführen einer Polymerisationsreaktion an dem Gemisch, um das thermoplastische Polymer zu bilden und sicherzustellen, dass das Kollagen gleichmäßig in dem thermoplastischen Polymer verteilt ist, um ein kollagenhaltiges Polymer zu bilden; und
(d) Granulieren des kollagenhaltigen Polymers, um das kollagenhaltigen Kunststoffmasterbatch zu bilden.

2. Kollagenhaltiger Vliesstoff nach Anspruch 1, wobei das zumindest eine Kunststoffmasterbatch ferner ein kollagenfreies Kunststoffmasterbatch umfasst, wobei die Zusammensetzung des kollagenfreien Kunststoffmasterbatches ein anderes thermoplastisches Polymer umfasst.

3. Kollagenhaltiger Vliesstoff nach Anspruch 2, wobei ein Gewichtsverhältnis des kollagenhaltigen Kunststoffmasterbatches nach Anspruch 1 zu dem kollagenfreien Kunststoffmasterbatch 1:1-99 beträgt.

4. Kollagenhaltiger Vliesstoff nach Anspruch 2, wobei das thermoplastische Polymer und das andere thermoplastische Polymer gleich sind.

5. Medizinische Vorrichtung, umfassend den kollagenhaltigen Vliesstoff nach Anspruch 1 oder 2.

6. Medizinische Vorrichtung nach Anspruch 5, wobei die medizinische Vorrichtung eine hämostatische Gaze oder einen Verband umfasst.

## Revendications

1. Tissu non tissé contenant du collagène, fabriqué à partir d'au moins un mélange maître de plastique par une technique de fusion-soufflage, dans lequel au moins un mélange maître de plastique comprend un mélange maître de plastique contenant du collagène dont la composition comprend :
un polymère thermoplastique, dans lequel le polymère thermoplastique est le polyéthylène téréphtalate ;
et
un collagène, dans lequel le collagène est uniformément réparti dans le polymère thermoplastique,
dans lequel dans le mélange maître de plastique contenant du collagène, une teneur en collagène est d'environ 1-50 % en poids, et
dans lequel le mélange maître de plastique contenant du collagène est formé par une méthode de polymérisation par fusion, dans laquelle la méthode de polymérisation par fusion comprend :
(a) effectuer une procédure de fusion sur au moins une matière première monomère constituant le polymère thermoplastique pour obtenir une matière première monomère fondue ;
(b) effectuer une procédure de chauffage et de mélange de la matière première monomère fondue et du collagène pour former un mélange ;
(c) effectuer une réaction de polymérisation sur le mélange pour former le polymère thermoplastique et s'assurer que le collagène est uniformément réparti dans le polymère thermoplastique pour former un polymère contenant du collagène ; et
(d) granuler le polymère contenant du collagène pour former le mélange maître de plastique contenant du collagène.

2. Tissu non tissé contenant du collagène selon la revendication 1, dans lequel le au moins un mélange maître de plastique comprend en outre un mélange maître de plastique sans collagène, dans lequel la composition du mélange maître de plastique sans collagène comprend un autre polymère thermoplastique.

3. Tissu non tissé contenant du collagène selon la revendication 2, dans lequel un rapport pondéral entre le mélange maître de plastique contenant du collagène selon la revendication 1 et le mélange maître de plastique sans collagène est de 1:1-99.

4. Tissu non tissé contenant du collagène selon la revendication 2, dans lequel le polymère thermoplastique et l'autre polymère thermoplastique sont identiques.

5. Appareil médical comprenant le tissu non tissé contenant du collagène selon la revendication 1 ou 2.

6. Appareil médical selon la revendication 5, dans lequel l'appareil médical comprend une gaze hémostatique ou un pansement.
